# EUROPEAN PATENT SPECIFICATION

(11) **EP 2 799 740 B1**
(45) Date of publication and mention of the grant of the patent: **24.07.2019**
(21) Application number: 14166592.7
(22) Date of filing: 30.04.2014
(51) Int. Cl.: F16H 25/20, A61M 5/178

(54) **Telescoping screw assembly and method for assembling a such**
Ausfahrbahre Spindelanordnung und Montageverfahren dafür
Ensemble télescopique à vis et procédé d'assemblage associé

(30) Priority: 30.04.2013 US 201313874085
(43) Date of publication of application: 05.11.2014
(73) Proprietor: Medimop Medical Projects Ltd., 43665 Ra'anana (IL)
(72) Inventor: Cabiri, Oz, 71799 Macabim (IL); Filman, Reuven Y., 42560 Netanya (IL); Bar-El, Yossi, 71947 Beit Arye (IL)
(74) Representative: Patentanwälte Vollmann Hemmer Lindfeld Partnerschaft mbB

(56) References cited:
- WO-A1-97/00091
- WO-A1-2010/089313
- DE-A1- 19 717 107
- US-A- 1 795 630
- US-A1- 2009 093 792

## Description

This application refers to US 2009/0093792 A1, US 2013/0190691 A1, WO 2011/090956, US 2011/0178463 A1 and to U.S. Application 13/874,017 entitled APPARATUSES FOR SECURING COMPONENTS OF A DRUG DELIVERY SYSTEM DURING TRANSPORT AND METHODS OF USING SAME the disclosures of all of which are incorporated herein by reference.

The present invention, in some embodiments thereof, relates to a high reliability telescoping assembly (TSA) and method of assembly for such and, more particularly, but not exclusively, to a unidirectional assembly method for a disengagement resistant TSA from molded parts.

U.S. Patent No. 7,967,795 to the instant applicant (Cabiri) discloses A cartridge interface assembly including a driving plunger including an outer shaft, and a driver including an inner shaft, the inner shaft mating with an intermediate shaft, the intermediate shaft mating with the outer shaft, so that the shafts are movable telescopically with respect to one another, wherein rotation of the driver causes the driving plunger to advance in a direction away from the driver.

U.S. Patent Application Publication No. 2009/0093792 to Gross discloses an apparatus for administering a substance to a subject. A vial contains the substance and a stopper is disposed within the vial and is slidably coupled to the vial. A first threaded element is (a) rotatable with respect to the vial and (b) substantially immobile proximally with respect to the vial during rotation of the first threaded element. A second threaded element is threadedly coupled to the first threaded element. At least a distal end of the second threaded element is substantially non-rotatable with respect to the vial, and the distal end of the second threaded element defines a coupling portion that couples the second threaded element to the stopper. The first threaded element, by rotating, linearly advances the stopper and at least the distal end of the second threaded element toward a distal end of the vial.

International Patent Application Publication No. WO/2011/090956 to the instant applicant (Cabiri) disclosed a cartridge interface assembly characterized by a driving plunger including an outer shaft, and a driver including an inner shaft movable telescopically with respect to the outer shaft, wherein rotation of the driver causes the driving plunger to advance in a direction away from the driver, and wherein the cartridge interface assembly is inserted in a cartridge in which a plunger is slidingly disposed, and rotation of the driver causes the driving plunger to advance distally in the cartridge until abutting against the plunger.

U.S. patent 8,157,769 to the present instant applicant (Cabiri) discloses A cartridge insertion assembly including apparatus with a pathway formed therein, a cartridge insertable into the pathway, the cartridge including a cartridge coupling element connectable to an activation mechanism disposed in the apparatus operative to cause a substance contained in the cartridge to be metered out of the cartridge, and a door pivoted to the apparatus that includes a door coupling element arranged with respect to the cartridge such that when the door is in a fully closed position, the door coupling element couples the cartridge coupling element with a coupling element of the activation mechanism.

U.S. patent 7225694 to Said, discloses a telescopic actuator that has a lead screw and one or more concentric or tiered screws. Each screw has one or more tangential interference stop features such as stop cogs. As the lead screw is rotated, it translates out of the concentric screws. As the lead screw reaches its maximum extension, a tangential interference stop feature on the lead screw tangentially contacts a tangential interference stop feature on the concentric screw with which the lead screw is threadably engaged. Upon tangential contact, the associated concentric screw rotates in unison with the lead screw. When there are additional concentric screws, as each concentric screw reaches its maximum extension, the system of tangential contacting of tangential interference stop features causes the other concentric screws to extend out in sequential fashion.

Additional background art includes U.S. patent 8220349, U.S. patent 6494005, U.S. patent 6435048, and U.S. patent 6382039.

WO 2010/089313 A1 discloses a telescoping assembly according to the preamble of claim 1 by describing a medicament delivery device comprising a housing, a telescopic piston rod assembly for driving a bung of a medicament container, and a drive mechanism for the telescopic piston rod assembly. The telescopic piston rod assembly comprises a plunger for driving a bung, a key for allowing predominantly axial movement between the plunger and the housing and an input drive telescopically coupled to the plunger and supported for rotational and axial movement relative the housing. The drive mechanism additionally comprises a transfer drive that operates to rotate the input drive. As the input drive rotates, it moves axially relative to the housing, expanding telescopically relative to the plunger.

One possible failure of known telescoping assemblies (TSA) is premature detaching and or disengaging of a threaded rod from a TSA of the delivery system. Typically, a rod may have a drive mechanism or an actuator on one end and a thread on the other end. A rod that is screwed to the assembly from the screw side may possibly be disengaged from the assembly in reverse direction. As a TSA is extended, the order of extension may sometimes become inverted causing the threads of one element to become disengaged before the assembly is completely extended. As result, the TSA may fail to extend completely resulting in failure to deliver a full dose of the drug.

An object of the invention relates to providing a TSA that resists disengagement and/or detaching of extension rods upon extension.

Although not in accordance with the present invention there is provided a method of assembling a telescoping screw including: threading a leading end of an inner rod into a rear end of a mid rod; also threading a leading end of the mid rod into a rear end of an outer rod so that the leading end of the inner rod is accessible from an leading end of the outer rod, and fastening a first end cap onto the leading end of the inner rod.

The method may further include extending the telescoping screw by further threading the inner rod to extend the leading end of the inner rod out of the leading end of the outer rod.

The method may further include driving the extending by rotating the first end cap.

The method may further include also fastening a second end cap onto the rear end of the outer rod.

The method may further include driving extension of the telescoping screw by rotating the second end cap.

The method may further include preventing an unthreading of the telescoping screw by providing a rotational stopper to limit unthreading rotation of the first end cap with respect to the outer rod

The rotational stopper may include an interference element between first end cap and to the outer rod.

The method may further include preventing an unthreading of the telescoping screw by providing a rotational stopper to limit unthreading rotation of the second end cap with respect to the inner rod.

The method may further include pushing a load by extending the telescopic screw.

The load may include a plunger of a syringe and the method further includes discharging a drug from the syringe by the pushing of the plunger.

The method may further include extending the telescoping screw by further threading the mid rod to extend a leading end of the mid rod out of the leading end of the outer rod.

The method may further include providing a fastener on the leading end of the inner rod.

The inner rod may be molded in one piece with the fastener.

The method may further include blocking over extension of the middle rod from the outer rod by a protrusion on the rear end of the middle rod.

The middle rod may be molded in one piece with the protrusion.

The method may further include blocking over extension of the inner rod from the mid rod by a protrusion on a rear end of the inner rod.

The inner rod may be molded in one piece with the protrusion.

The method may further include supporting the telescoping screw against an inside wall of a syringe via a shoulder.

According to the present invention there is provided a telescoping assembly according to claim 1.

According to some embodiments of the invention, each of the inner rod, mid rod and outer rod is molded in a single piece.

According to the invention, the telescoping assembly further includes a first end cap including a matching fastener for fastening to the rear fastener.

According to some embodiments of the invention, the inner rod further includes a leading fastener.

According to some embodiments of the invention, the telescoping assembly further includes a second end cap including a matching fastener for fastening to the leading fastener.

According to the invention, the telescoping assembly further includes a stopper for preventing dethreading of telescoping assembly, the stopper including an interference element on the second end cap for preventing rotation in a contracting direction of the outer rod with respect to the second end cap.

According to some embodiments of the invention, the telescoping assembly further includes a shoulder fitting into a bore of a syringe and rotatable with respect to the syringe the shoulder supporting the telescoping assembly.

According to some embodiments of the invention, the inner rod, the mid rod and the outer rod are all threaded in a first direction, and the telescoping assembly further includes an end cap with a thread in an opposite direction.

According to some embodiments of the invention, the respective pitch of threading of each of the inner rod, the mid rod and the outer rod is adjusted to produce the same magnitude of linear motion per rotation.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart showing a method of assembling a telescoping assembly (TSA), in accordance with an exemplary embodiment of the invention;
FIGs. 2A-D are schematic cross-sectional illustrations of a TSA in an extended state, a contracted state, an exploded view, and a reverse extended state, in accordance with an exemplary embodiment of the invention;
FIG. 2E is schematic cross-sectional illustration of an alternative embodiment of a TSA in an a reverse extended state, in accordance with an exemplary embodiment of the invention;
FIGs. 3A-B are perspective views of an alternative embodiment of a TSA in contracted, extended states respectively, in accordance with an exemplary embodiment of the invention;
FIGs. 3C-D are cross sectional views of an alternative embodiment of a TSA installed in a syringe in contracted and extended states respectively, in accordance with an exemplary embodiment of the invention;
FIG. 3E is an exploded reverse extended view of an alternative embodiment of a TSA, in accordance with an exemplary embodiment of the invention;
FIGs. 3F-M are perspective views of steps of assembling an alternative embodiment of a TSA, in accordance with an exemplary embodiment of the invention;
FIGs. 4A-B are perspective views of an alternative embodiment for a TSA including an alternative end cap and stopper, in accordance with an exemplary embodiment of the invention;
FIGs. 5A-B are cross sectional views of an another alternative embodiment of a TSA inserted into a syringe in a contracted state, in accordance with an exemplary embodiment of the invention;
FIG. 5C is cutaway view of another alternative embodiment of a TSA inserted into a syringe, in accordance with an exemplary embodiment of the invention;
FIG. 6 is a cutaway view of an alternative embodiment of a TSA with a counter sunk shoulder support, in accordance with an exemplary embodiment of the invention; and
FIG. 7 is a state diagram illustrating states of a TSA, in accordance with an exemplary embodiment of the invention.

The present invention, in some embodiments thereof, relates to a high reliability telescoping assembly (TSA) and method of assembly for such and, more particularly, but not exclusively, to a unidirectional assembly method for a disengagement resistant TSA from molded parts.

### 1. Simple assembly of a TSA

An aspect of some embodiments of the invention relates to providing a TSA that is assembled in a simple manner. Optionally, assembly may be unidirectional. Unidirectional assembly may include, for example, insertion of all or most extension rods from the same end of the TSA. Unidirectional assembly may include, for example, threading all extension rods the same direction. Optionally, assembly may be accomplished without reversing orientation of the assembly during assembly and/or adding other complimentary work such as welding, riveting, plastic deformation Etc.

In some embodiments, a series of extension rods may be threaded together. For example for each rod in the series, a leading end of an interior rod may be threaded may be threaded through into a rear end of a more exterior rod. As used herein, the term/phrase leading end means the end of a TSA from which the inner rod projects in the extended state. As used herein, the term/phrase rear end means the end of a TSA from which the outer rod projects in the extended state. As used herein, the term/phrase threading means screwing the more interior rod towards the leading direction. As used herein, the term/phrase de-threading means screwing the more interior rod towards the rear of the outer rod. Optionally, the leading end of the most inner rod may include a fastener and/or the rear end of the most external rod may include a fastener.

Developing an effective drug therapy and treatment includes more than simply finding an effective molecule. It includes combination of a safe drug within a suitable container and/or delivery system.

One possible failure is premature detaching and or disengaging of a threaded rod from a TSA of the delivery system. Typically, a rod may have a drive mechanism or an actuator on one end and a thread on the other end. A rod that is screwed to the assembly from the screw side may possibly be disengaged from the assembly in reverse direction. As a TSA is extended, the order of extension may sometimes become inverted causing the threads of one element to become disengaged before the assembly is completely extended. As result, the TSA may fail to extend completely resulting in failure to deliver a full dose of the drug.

Another source of failure of a TSA is errors and/or imprecision in the TSA assembly process. For example, a complex assembly processes and/or low precision modification of the parts during assembly may decrease the reliability of a TSA.

### 2. Disengagement resistant TSA

An aspect of some embodiments of the invention relates to providing a TSA that resists disengagement and/or detaching of extension rods upon extension. For example, an internal rod of the TSA may include a flange on its rear end. The flange may prevent disengagement from a more outer rod. Alternatively or additional the flange may be replaced by a protrusion of a different geometry.

### 3. Use of molded parts

An aspect of some embodiments of the invention relates to providing a TSA that is assembled from molded parts. In some embodiments, molding provides highly precise part geometries. Molded parts may optionally be assembled with minimal modifications during assembly. For example, the assembly of the TSA may be include minimal or no adhesion of parts, and/or changing of part geometries by heat and/or ultrasonic means and/or by force (for example by crimping). The molded parts may optionally include features to facilitate proper orientation. The molded parts may optionally include built in connectors and/or fasteners (for example snaps, latches, catches, hooks, clasps and the like). In some embodiments that parts may be molded of plastic. For example plastic may include low friction materials. Example of such materials includes for example CELANEX® (for example 2405 MT) available from TICONA and Delrin® (for example 100AL NC010) by DuPont™.

In some embodiments an internal rod may be molded in a single piece with the rear flange and/or projections. The flange and/or projection may optionally impeded unintentional disengagement of the rod. In some embodiments a part may be molded in a single piece with a fastener. In some embodiments a part may be molded in a single piece with a stopper.

### 4. Stoppers

An aspect of some embodiments of the invention relates to a stopper that may stop rotation of a TSA. For example a protrusion molded into an end cap may interact with a corresponding protrusion molded into an adjoining end of an extension rod to stop rotation of the rod and/or of a plurality of rods at a predefined point. For example, the stopper may prevent thread lock and or dethreading of the TSA after the TSA reaches a contracted state

### 5. Shoulder Support

An aspect of some embodiments of the invention relates to an end cap shoulder which supply support to a TSA in a syringe. For example a driver may include a shoulder which is inserted into the bore of a syringe to provide support for the outer end of the TSA. The shoulder may optionally rotate in the syringe bore. Optionally, the space for the shoulder may be counter sunk into the syringe flange. Countersinking the shoulder may save space in the syringe bore for the TSA.

### Exemplary Embodiments

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### 1. Reverse assembly and installation of end caps

In some embodiments, some or all of the extension rods of the assembly may optionally be supplied disassembled from the end caps. For example, the some or all of the rods and/or end caps may include fasteners. The extension rods may optionally be supplied with flanges inhibiting disassembly due to overextension. For example, an internal rod may have a flange on a rear end. The flanges and/or fasteners may optionally be intrinsic. For example, the rods and/or caps may be molded in a single piece with the fasteners and/or flanges.

In some embodiments, some or all of the extension rods assembled together by reverse extension. For example, a leading end of an internal rod may be threaded into a rear end of a more external rod. For example, the internal rod may be threaded from a disassembled (reverse extended) position through its contracted position out the leading end of a mating rod to an extended position. In some embodiments, threading from the reverse extended position may be necessary because flanges which prevent the rods from disattaching in the extended state may also prevent attaching the rods in the extended state and threading them to the contracted state.

In some embodiments, a fastener may be supplied on a leading end of an inner rod. Once the leading end of an inner rod extends beyond the mating rod, an end cap (for example a driver and/or an actuator) may be fastened to the fastener. Optionally a stopper may be supplied. For example the stopper may block dethreading back to the reverse extended position and/or may prevent thread lock resulting from collision between a rod and an end cap.

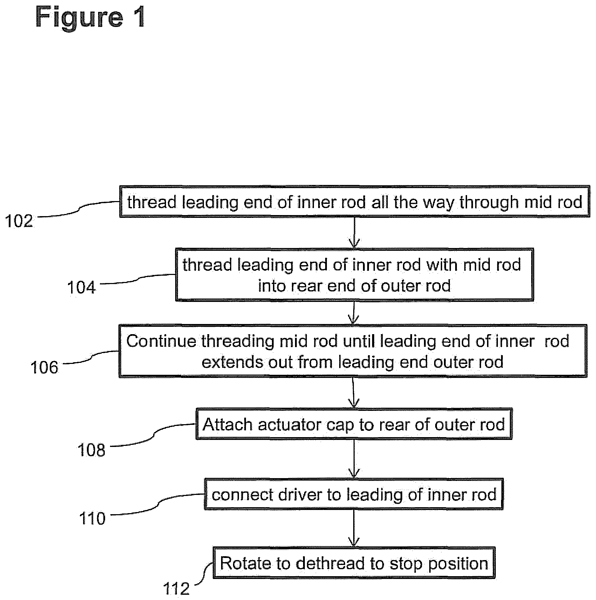

Referring now to the drawings, Figure 1 illustrates an exemplary method for assembling a TSA. In some embodiments, the leading end of an inner rod may be threaded 102 into the rear end of a mid rod. Optionally the leading end of the inner rod may be threaded all the way through a mid rod until the leading end of the inner rod protrudes from the leading end of the mid rod.

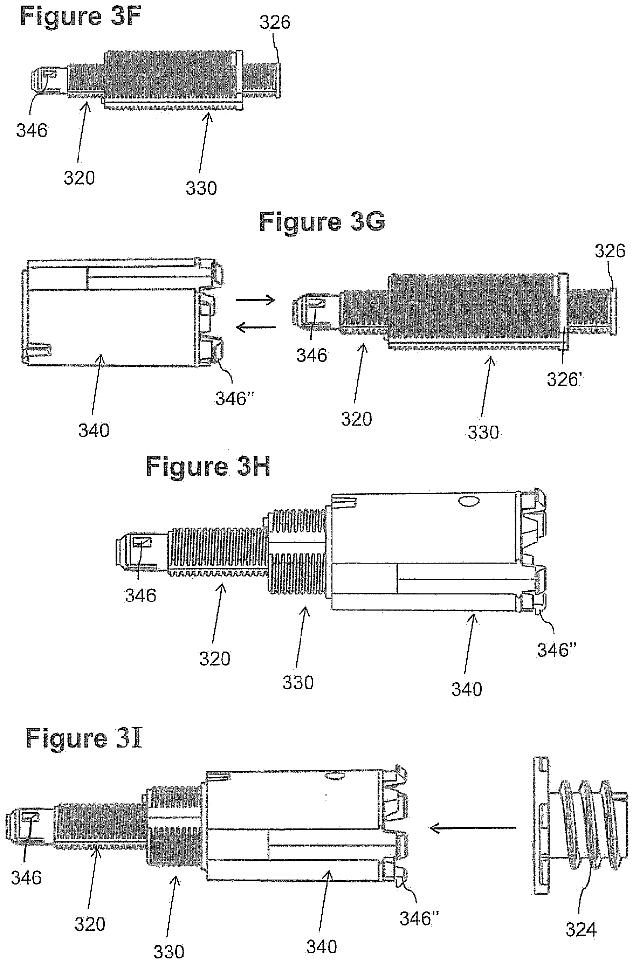

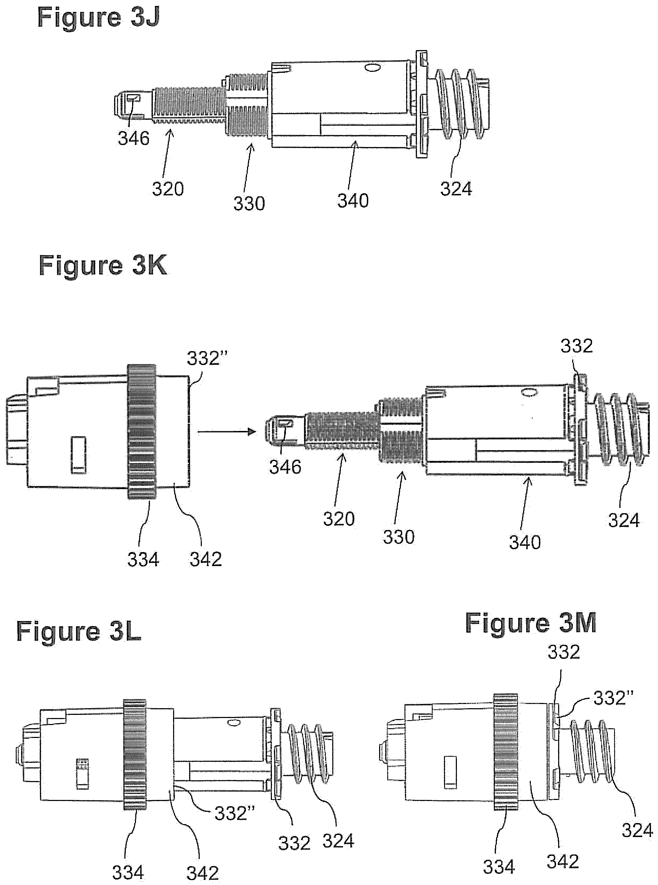

In some embodiments, the leading end of the inner rod along with the middle rod may be threaded 104 into a rear end of an outer rod. Threading 104 be continued 106 until the leading end of the inner rod protrudes out the leading end of the outer rod. Optionally the assembly may include only two rods and/or more than three rods (for example four, five, six or more rods). In some embodiments, regardless of the number of rods, the assembly of rods may have a fastener of the inner rod protruding from the leading end and a fastener of an outer rod protruding from the rear end (for example see Fig. 3H).

In some embodiments, an actuator cap (for example cap 324 of Fig. 3I) may be attached 108 to the fastener of the outer rod. For example, an actuator cap may include a syringe plunger and/or a fitting to attach to a syringe plunger. Examples of syringe plungers actuated by telescopic assemblies can be found for example in International Patent Application Publication No. WO/2011/090956 by the instant applicant (Cabiri) and/or U.S. Patent Application Publication No. 2009/0093792 to Gross which are herein incorporated in their entirety by reference.

In some embodiments, a driver cap (for example cap 334 of Fig. 3K) may be attached 110 to the fastener of the inner rod. For example, a driver cap may include a gear for rotating the inner rod in order to extend and/or contract the TSA.

In some embodiments, after assembly, the TSA may be dethreaded 112 to a stop position. Optionally, the TSA may include one or more stoppers. For example a pair of stoppers on the two end caps (for example the drive cap and the actuator cap) may meet at a predefined point in the contraction of the TSA and prevent further relative rotation in the dethreading direction of the inner rod with respect to the outer rod thereby preventing further contraction (for example see stoppers 332 and 332" of Figs. 3K and 3M).

### 2. Components of a TSA and their interconnection

In some embodiments, a TSA may be simple assembled unidirectionally from a reverse extended position. The assembled TSA may optionally resist dis-assembly by extension.

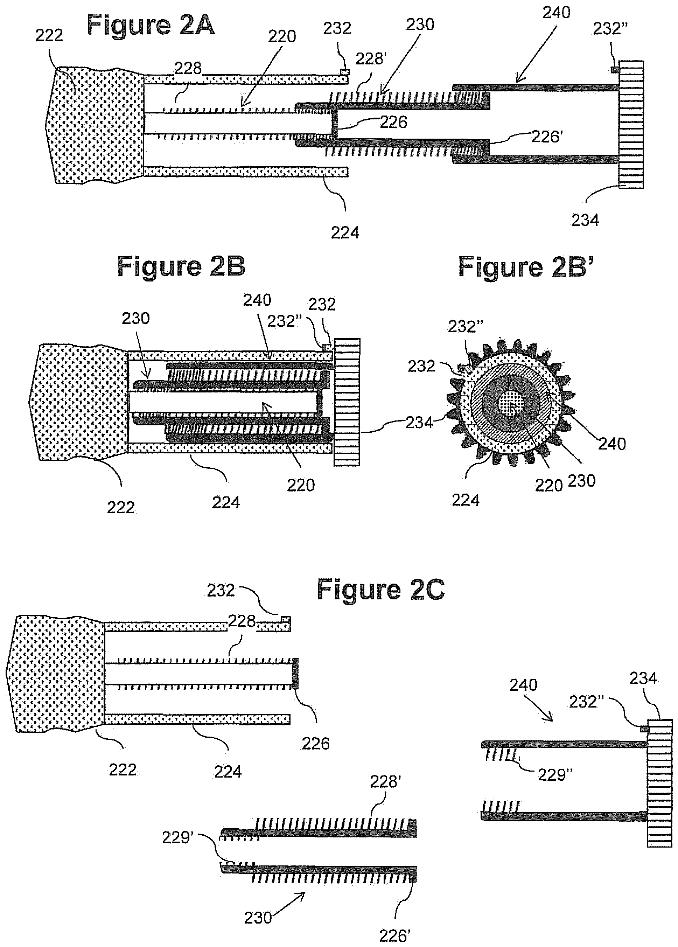

Referring now to the drawings, Figure 2A illustrates an exemplary schematic representation of a TSA in an extended state. One end the assembly may, for example, be connected to an actuator mount 224 and/or an actuator 222 (for example actuator 222 may include a plunger for discharging medicine from a syringe). The other end may, optionally, be connected to a driver 234 (for example driver 234 may include a gear to rotate a threaded rod (for example an outer rod 240) thereby extending and/or contracting the TSA).

In some embodiments expansion and contraction of the TSA may be effected by a series of threaded rods. For example, an inner rod 220 may be threaded into a rear end of a mid rod 230 which may optionally be threaded into a rear end of an outer rod 240. In the exemplary embodiment of Figs. 2A-E, rods 220, 230, and/or 240 may optionally include interior threads (for example threads 229' and/or 229" (see Fig. 2C)) and/or exterior threads (for example threads 228 and/or 228' (see Fig. 2C)).

In some embodiments a rod may include a member to prevent disengagement of the rod during extension of the TSA. For example inner rod 220 includes a rear flange 226 and mid rod 230 includes a rear flange 226'. When rod 220 reaches full extension, flange 226 contacts interior threads 229' of mid rod 230 preventing further extension (see Fig. 2A). When rod 230 reaches full extension, flange 226' contacts interior threads 229" of outer rod 230 preventing further extension (see Fig. 2A). Alternatively or additionally a flange (for example flange 226 and/or 226') may be include a protrusion of any geometry and not necessarily and annular ring.

In some embodiments, a TSA may include stop element. For example, Figs. 2B illustrate a cutaway side view and a cutaway end on view of the TSA of Figs. 2A-D in a fully contracted state. A protrusions 232 on driver 234 contacts a matching protrusion 232" on actuator mount 224 inhibiting further relative rotation in the dethreading direction between driver 234 and actuator mount 224. Optionally thereby further de-threading may be prevented between inner rod 220 and outer rod 240.

Fig. 2C illustrates the exemplary TSA of Figs. 2A-D with rods 220, 230, and 240 disassembled. Flanges 226 and 226' which prevent disengagement of rods may also prevent assembly of rods in the extended state. Driver 234 and end cap 232 may prevent assembly in the opposite direction.

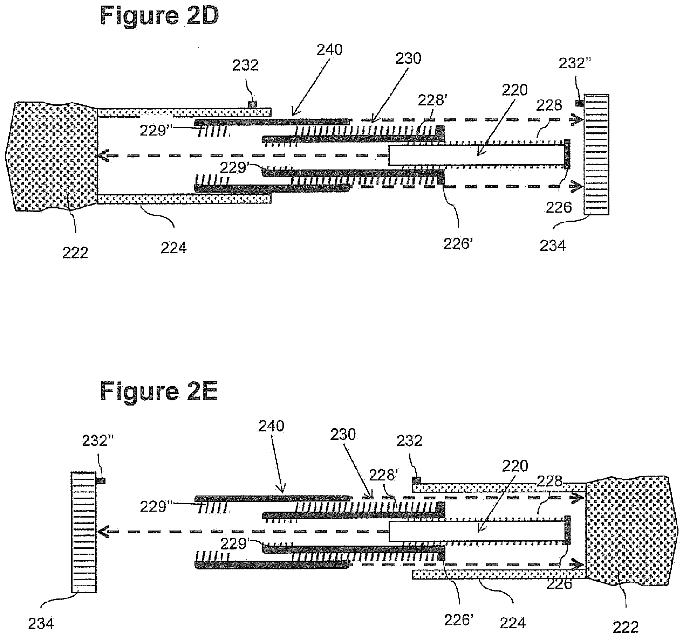

Fig 2D illustrates the exemplary embodiment of a TSA of Figs. 2A-D in a reverse extended configuration (extension rods 220, 230 and 240 are in the opposite order of extended state of Fig. 2A). In some embodiments, assembly of the TSA may start from the reverse extended state. Assembly may optionally proceed unidirectionally, some and/or all of the extension rods being sequentially inserted into the assembly in the same direction. For example, from the reverse extended state, a leading end of inner rod 220 may be threaded through the rear end of mid rod 230.

For example the leading end mid rod 230 and inner rod 220, may be threaded in the same direction together through the rear end of outer rod 240.

In some embodiments, after threading together the extension rods, end caps may be attached. For example, actuator mount 224 and/or actuator 222 may be attached to a leading end of inner rod 220 (as illustrated by the broken arrow in the left side of Fig. 2D). For example, after threading together the extension rods (for example rods 220, 230, and 240) an end cap (for example driver 234) may be attached to a rear end of outer rod 240 (as illustrated, for example, by the dual dashed arrows on the right side of Fig. 2D).

Fig. 2E illustrates assembly of an alternative exemplary embodiment of a TSA in the reverse extended state. The exemplary embodiment of Fig. 2E has end caps reversed (actuator 222 is connected to outer rod 240 and drive 234 is connected to inner rod 220) with respect to the embodiment of Fig. 2D.

### 3. An example of assembly of an external TSA with snap on end caps and shoulder support

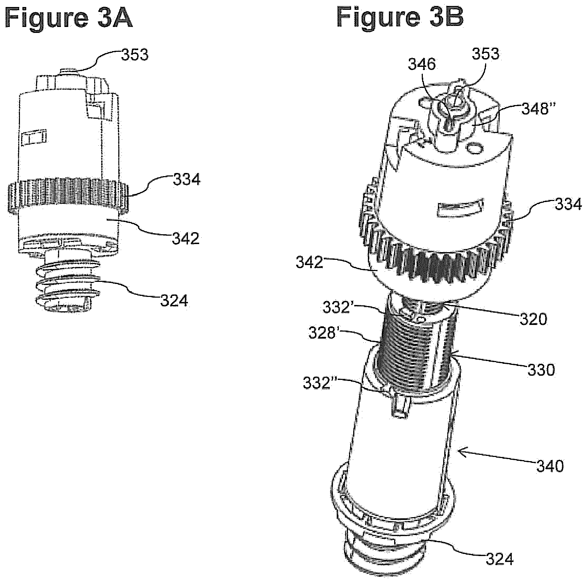

Figs. 3A-M illustrate an exemplary embodiment of an out of syringe unidirectional assembled TSA having optional snap on end caps and a shoulder support. Snap on end caps may optionally allow connecting end caps without modifying, gluing, or melting parts during assembly. A TSA may optionally include a shoulder support. The shoulder support may be inserted into a syringe bore. For example the shoulder may add to the mechanical stability of the telescoping assembly during operation. The shoulder may optionally hold an extension rod in proper relation to a bore (for example of a syringe). The shoulder may optionally rotate with respect to the syringe bore.

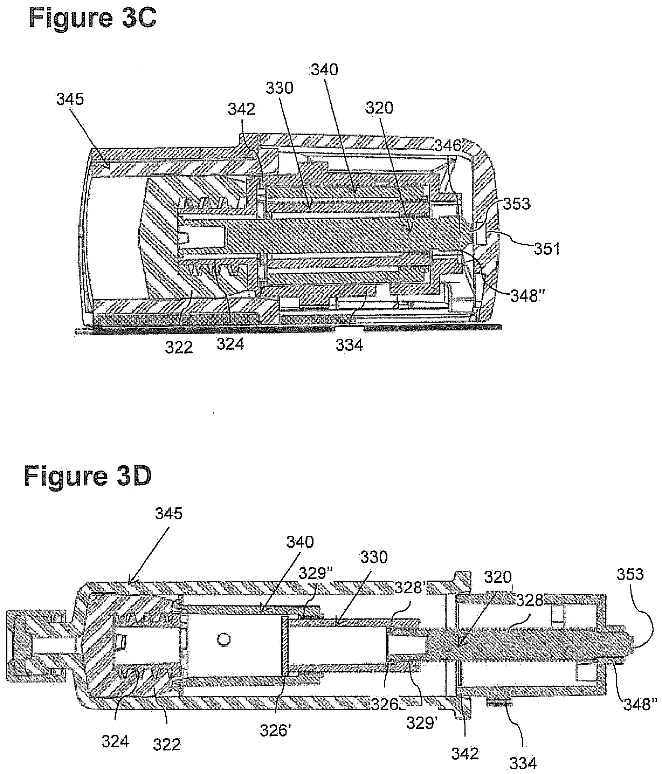

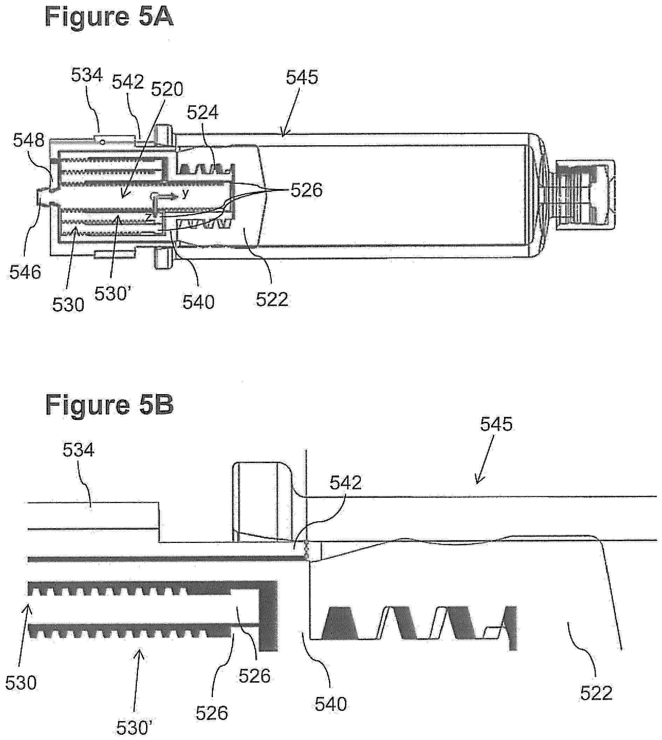

Referring now to the drawings, Fig. 3A illustrates perspective views of an alternative exemplary embodiment of a PSA in a contracted state. The two end caps can be seen; including for example a driver 334 and/or an actuator mount 324. The actuator mount may optionally include a screw thread to attach to a syringe plunger. The driver may optionally include a shoulder 342 configured to fit into a syringe bore as seen for example in Figs. 3C,D and 5A,B. The shoulder may support the TSA against movements (for example sideways translation) caused by forces on the driver.

Fig. 3B illustrates a perspective view of an alternative exemplary embodiment of a TSA in an extended state. Along with the end caps, extension rods including an inner rod 320, a mid rod 330 and an outer rod 340, are visible in Fig. 3B. External screw 328' is also shown on the mid rod. Also shown in Fig. 3B are optional stoppers including projections 332' and 332" which fit into recesses (not shown) in driver 334 to stop relative rotation of outer rod 340 and/or mid rod 330 with respect to driver 334 and/or inner rod 320 as the TSA reaches a contracted state.

Fig. 3C illustrates a cutaway view of an alternative exemplary embodiment of a TSA installed into a syringe 345 in a contracted state. Shoulder 342 is at least partially inserted into the bore of syringe 345. In the example of Fig. 3C, in the contracted state, most of the length of the TSA projects out of syringe 345. For example, in the example of Fig. 3C, mid rod 330 and/or outer rod 340 sit entirely outside syringe 345. For example, in the example of Fig. 3C, inner rod 320 is mostly outside of syringe 345 with a small portion extending inside syringe 345.

In the example of Fig. 3C, support for the TSA is supplied on the distal end by shoulder 342 which extends into the bore of syringe 345 and is supported by the inner walls of the syringe. Optionally, syringe 345 may and/or shoulder 342 (and/or all of driver 334) may be made of low friction materials. For example, the inner walls of syringe 345 may support shoulder 342 against lateral forces (preventing lateral translation). Nevertheless, shoulder 342 may optionally be able to rotate within the syringe bore.

In the example of Fig. 3C, support for the TSA is supplied on the proximal end by an axel 353 extending from the proximal end of inner rod 320 out of driver 334 into a hub 351. Hub 351 may for example, be part of a door of a patch injector for example as described in U.S. patent 8,157,769 to the instant applicant (Cabiri) which is herein incorporated in their entirety by reference. Optionally, hub 351 and/or axel 353 (and/or all of inner rod 320) may be made of low friction materials. For example, hub 351 may support axel 353 against lateral forces (preventing lateral translation). Nevertheless, axel 353 may optionally be able to rotate within hub 351.

Referring now to the figures, Fig. 3D is a cutaway view of an alternative exemplary embodiment of a TSA installed into a syringe in an extended state. Flanges 326 and 326' are illustrated on rods 320 and 330 respectively. Flanges 326 and 326' may optionally restrain respective extension rods 320 and 330 from disengaging upon extension. For example flange 326 may prevent extension rod 320 from disengaging from inner teeth 329' of extension rod 330 upon extension. For example flange 326' may prevent extension rod 320 from disengaging from inner teeth 329" of extension rod 330 upon extension. In Figs. 3C and 3D, actuator mount 324 is screwed into a plunger 322.

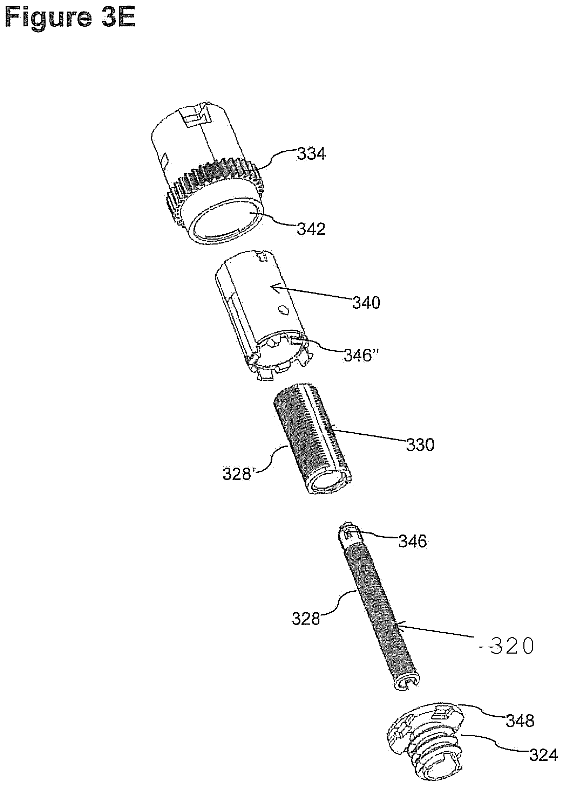

Referring now to the figures, Figs. 3E-M illustrate assembly of an alternative exemplary embodiment of a TSA. Fig. 3E illustrates an exploded view of the exemplary embodiment of a TSA in a reverse extended configuration ready for assembly. Optionally, in the embodiment of Fig. 3E, fasteners 346 may be supplied on inner rod 320 for attachment to a matching fastener (for example a hole 348") in a driver 334. Optionally, in the embodiment of Fig. 3E, fasteners 346" may be supplied on outer rod 340 for attachment to fasteners 348 of actuator mount 324.

In the illustrative example of Figs. 3A-M, assembly of the TSA may optionally start by threading inner rod 320 into mid rod 330, for example as illustrated in Fig. 3F. Optionally, starting from the reverse extended configuration (for example as illustrate in Fig. 3E) the leading end of inner rod 320 may be threaded all the way through mid rod 330 until it protrudes from the leading end of mid rod 330 as illustrated for example in Fig 3F.

In the illustrative example of Figs. 3A-M, after threaded inner rod 320 through mid rod 330, the combination may optionally be threading into outer rod 340, for example as illustrated in Fig. 3G. Optionally, threading may continue until the leading end of inner rod 320 protrudes out the far end of outer rod 340 as illustrated for example in Fig 3H. Optionally, all rods may be assembled by threading in the same direction, from the same end of the assembly.

In the illustrative example of Figs. 3A-M, after threading mid rod 330 through outer rod 340, an end cap (for example actuator mount 324) may be fastened to the rear end of outer rod 340, for example as illustrated in Fig. 3I. Optionally, fasteners may be molded into the cap and/or rod. For example, fasteners may include hooks (for example fasteners 346" and/or holes and/or eyes (for example fastener 348 and/or 348"). For example, in the exemplary embodiment of Figs. 3A-M fasteners 346" permanently latches into fasteners 348. For example, after fastening outer rod 340 to actuator mount 324, preventing actuator mount 324 from rotating prevents outer rod 340 from rotating. Fasteners may optionally include for example flanges (for example fasteners 346), locks, rivets, buckles, clips, snaps, latches, hinges, plugs, spacers, pins, grommets, nuts, bushings, clamps, clasps and/or screws. Fasteners may be permanent and/or reversible. Fig. 3J illustrates the exemplary embodiment of Figs. 3A-M after fastening actuator mount 324 to outer rod 340.

In the illustrative example of Figs. 3A-M, after fastening actuator mount 324 to outer rod 340, an end cap (for example driver 334) may be fastened to the end of outer inner rod 320, for example as illustrated in Fig. 3K. For example, in the exemplary embodiment of Figs. 3A-M fastener 346 permanently latches into a hole 348" of driver 334. For example, after fastening inner rod 320 to driver 334, rotating driver 334 also rotates inner rod 320. Fig. 3J illustrates the exemplary embodiment of Figs. 3A-M after fastening driver 334 to inner rod 320.

In the illustrative example of Figs. 3A-M, after fastening driver 334 to inner rod 320, the TSA may be dethreaded together into the contracted position as illustrated in Fig. 3M. A pair of stoppers 332, 332" may stop further dethreading of driver 334 with respect to actuator cap 324 once the TSA reaches the fully closed state.

### 4. Alternative embodiments

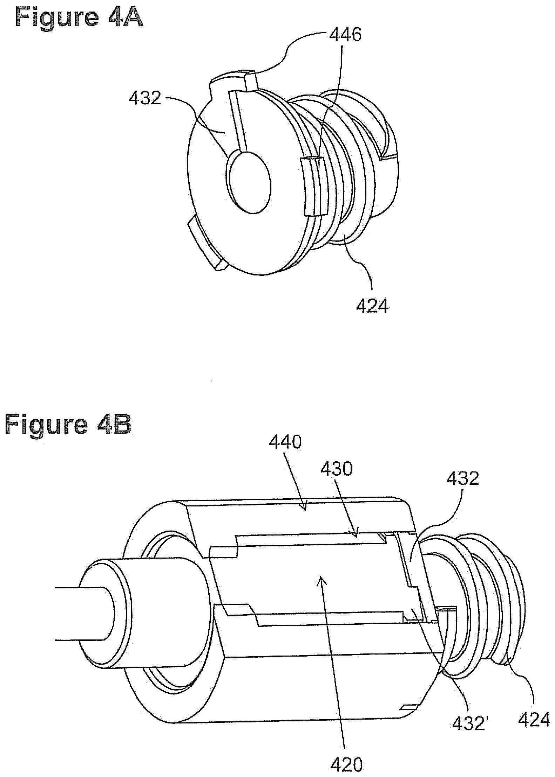

Referring now to the figures, Figs. 4A-B illustrate an alternative embodiment of a TSA. In the exemplary embodiment of Figs. 4A-B an actuator mount 424 serves as an end cap to an outer rod 440. Actuator mount 424, includes fasteners 446 that protrude sideways to snap into holes in the sides of outer rod 440. The exemplary embodiment of Figs. 4A-B includes an inner rod 420 and a mid rod 430. Actuator mount 424, includes an exemplary stopper 432 which includes a ridge on the inner face of actuator mount 424. When the ridge of stopper 432 meets a ridge 432' on the base of an inner rod 420, further dethreading is prevented between inner rod 420 and actuator mount 424. Thereby further contraction of the TSA is stopped. Optionally, stoppers 432, 432', 232, 232" may stop contraction of the TSA without putting significant longitudinal force between a rod and the end piece.

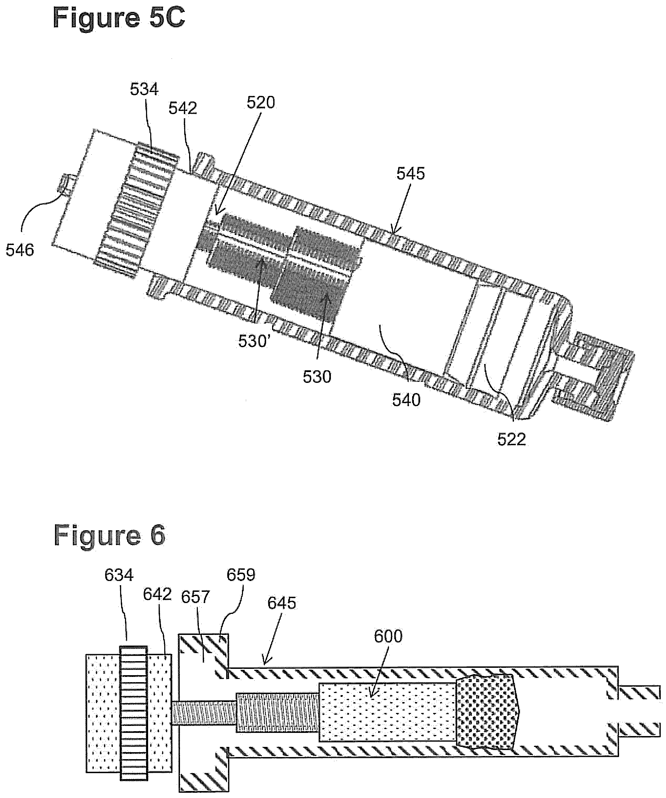

Figs. 5A-C illustrate an alternative exemplary embodiment of a TSA. Figs. 5A-B illustrate respective large scale and close up cutaway views of the alternative embodiment of the TSA installed into a syringe 545 in a contracted state. The embodiment of Figs. 5A-C optionally includes four threaded extension rods, an inner rod 520, two mid rods 530, 530' and an outer rod 540. Each rod includes an optional flange 526. Outer rod 540 is optionally integrally formed with an actuator mount 524 which is screwed into a plunger 522. Inner rod 520 includes an optional fastener 546 which snaps into a hole 548 in a driver 542. Driver 534 includes an optional shoulder support 542 which is illustrated inserted into the syringe bore. Fig. 5C illustrates a perspective see through view of the alternative embodiment of a TSA inserted into syringe 545.

Fig. 6 illustrates an alternative embodiment of a TSA 600 inserted into a syringe 645. The bore of syringe 645 includes an optional counter sunk cavity 657. For example, a shoulder 642 of a driver 634 may fit into counter sunk cavity. Counter sunk cavity 657 may extend into a flange 659 of syringe 645.

In some embodiments counter sunk cavity 657 may be wider than the bore of syringe 645. Optionally shoulder 642 may be wider than the bore of the syringe. Optionally making shoulder 642 wider than the syringe bore may make it possible to make more complete use of the bore for expansion rods of TSA 600.

### 5. State diagram of a TSA

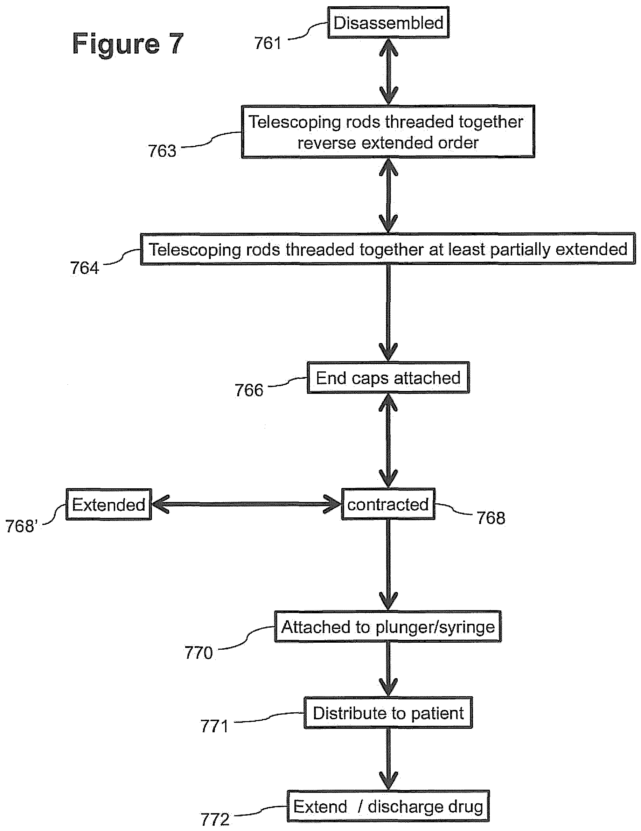

Referring now to the figures, Fig. 7 is a state diagram illustrating exemplary states of a TSA. A TSA may optionally be molded of parts in a disassembled 761 state. The parts may, for example include threaded telescoping rods. In assembly of the TSA, the rods may optionally be threaded together from a reverse extended configuration 763. The Rods may be reversibly threaded together further into a contracted and/or partially and/or fully extended 764 state.

In some embodiments, one or more end caps may be attached 766 to the extension rods. Attachment of end caps may optionally be irreversible (alternatively or additionally attachment 766 of end caps may be reversible).

In some embodiments, the end caps may inhibit return of the telescoping assembly to the reverse extended state and/or separating of the extension rods. For example, the end caps may block dethreading of the extension rods beyond the contracted state to the reverse extended state and eventually detached state. Alternatively or additionally, an end cap may include a stopper the stops movement in a certain direction of an extension rod at a certain point. For example, a stopper may prevent dethreading when an extension bar and/or the TSA reach a fully contracted state. Alternatively or additionally, the stopper may prevent further threading into super extension when the TSA reaches a fully extended state.

In some embodiments, with extension rods assembled and the end caps attached 766 the TSA can be reversibly extended 768' and/or contracted 768.

In some embodiments, the TSA will be contracted 768 and attached 770 to the plunger of a syringe. Optionally attachment 770 to a syringe may be irreversible. Alternatively or additionally attachment 700 to a syringe may be reversible.

In some embodiments, a syringe and attached TSA will be distributed 771 to a patient, for example as components of a patch injector. The patent may activate the injector, extending 772 the TSA to inject a drug. In some embodiments, extension of the TSA for discharging the drug will be irreversible. For example, after discharging the drug to the patch injector and/or the TSA and/or the syringe may be disposable. Alternatively or additionally, some or all components of a patch injector and/or TSA and/or syringe may be reusable.

### 6. Threading

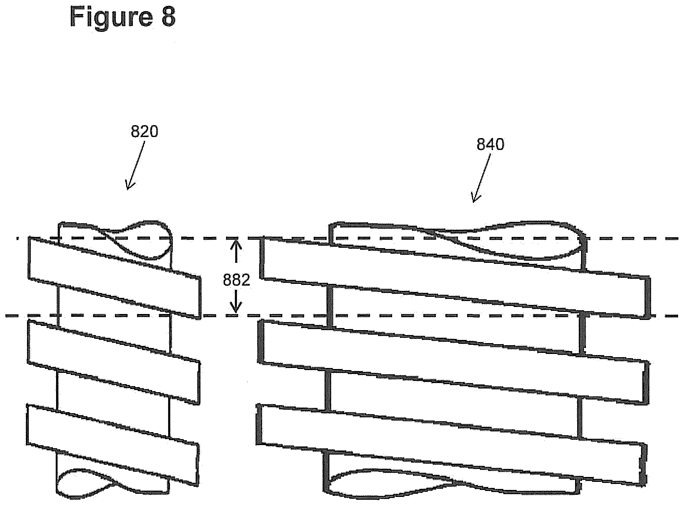

Fig. 8 illustrates threading of an inner rod 820 and an outer rod 840 in accordance with an exemplary embodiment of the invention. The pitch of the threads of each rod is adjusted so that the each rod moves the same linear distance per rotation (for example twice the distance 882) regardless of the diameter of the rod. Optionally, the linear movement of the syringe plunger and/or the quantity of medicine discharged per rotation of the driver is fixed regardless of which extension rod is active.

Extension rods 820 and 840 of Fig. 8 optionally all have a thread oriented in a single direction (for example in Fig. 8 rods 820, 840 have left hand screws). Optionally, an actuator cap (for example cap 324 of Fig. 3I) may have an opposite oriented screw thread (for example a right hand screw). Optionally, when the telescoping assembly is extended, the torque between the plunger and end cap will tend to tighten the connection between the plunger and the cap.

It is expected that during the life of a patent maturing from this application many relevant fasteners and/or other relevant parts will be developed and the scope of the terms fattener and/or other terms are intended to include all such new technologies *a priori.*

As used herein the term "about" refers to ± 5 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A telescoping assembly comprising:
an outer rod (240, 340, 440, 540, 840) including
a rear fastener (346") and
an internal thread (329") proximate a leading end thereof;
a mid rod (330) including an external thread (328") fitting said internal thread of said outer rod (240, 340, 440, 540, 840),
**characterized by**
the mid rod including a rear flange (226', 326') slidable through a portion of the outer rod (240, 340, 440, 540, 840) and being wider than said internal thread of said outer rod (240, 340, 440, 540, 840) and
an internal thread proximate a leading end thereof; and
the telescoping assembly further comprising
an inner rod (320, 420) including, an external thread (328) fitting said internal thread of said mid rod a rear flange (226, 326) slidable through a portion of the mid rod and being wider than said internal thread of said mid rod;
a first end cap (324, 424) including a matching fastener (348, 446) for fastening to said rear fastener (346") of the outer rod (340, 440); and
a stopper (432) for preventing dethreading of the telescoping assembly, said stopper (432) including an interference element on said first end cap (324, 424) for preventing rotation in a contracting direction of said inner rod with respect to said first end cap (324, 424)

2. The telescoping assembly of claim 1, wherein each of said inner rod, mid rod and outer rod (240, 340, 440, 540, 840) is moulded in a single piece.

3. The telescoping assembly according to any of the claims 1 or 2, wherein said inner rod further comprises a leading fastener (346).

4. The telescoping assembly according to any of the claims 1 to 3, wherein said telescoping assembly includes a shoulder fitting into a bore of a syringe (345, 645) and rotatable with respect to said syringe said shoulder supporting said telescoping assembly.

5. The telescoping assembly according to any of the claims 1 to 4, wherein said inner rod, said mid rod and said outer rod (240, 340, 440, 540, 840) are all threaded in a first orientation further including an end cap with a thread in an opposite orientation.

6. The telescoping assembly according to any of the claims 1 to 5, wherein a respective pitch of threading of each of said inner rod, said mid rod and said outer rod (240, 340, 440, 540, 840) is adjusted to produce the same magnitude of linear motion per rotation.

## Patentansprüche

1. Teleskopartige Anordnung, umfassend:
einen äußeren Stab (240, 340, 440, 540, 840), umfassend
ein hinteres Befestigungsmittel (346") und
ein Innengewinde (329") nahe einem Vorderende davon;
einen mittleren Stab (330), umfassend
ein Außengewinde (328"), das zu dem Innengewinde des äußeren Stabes (240, 340, 440, 540, 840) passt,
**dadurch gekennzeichnet, dass**
der mittlere Stab einen hinteren Flansch (226', 326'), der durch einen Abschnitt des äußeren Stabes (240, 340, 440, 540, 840) verschiebbar und breiter als das Innengewinde des äußeren Stabes (240, 340, 440, 540, 840) ist, und ein Innengewinde nahe einem Vorderende davon umfasst; und
wobei die teleskopartige Anordnung ferner umfasst:
einen inneren Stab (320, 420), umfassend:
ein Außengewinde (328), das zu dem Innengewinde des mittleren Stabes passt,
einen hinteren Flansch (226, 326), der durch einen Abschnitt des mittleren Stabes verschiebbar und breiter als das Innengewinde des mittleren Stabes ist;
eine erste Endkappe (324, 424), umfassend ein passendes Befestigungsmittel (348, 446) zum Befestigen an dem hinteren Befestigungsmittel (346") des äußeren Stabes (340, 440); und
einen Anschlag (432) zum Verhindern des Außergewindeeingriffbringens der teleskopartigen Anordnung, wobei der Anschlag (432) ein Zwischenelement an der ersten Endkappe (324, 424) zum Verhindern einer Drehung in einer Zusammenziehrichtung des inneren Stabes in Bezug auf die erste Endkappe (324, 424) umfasst.

2. Teleskopartige Anordnung nach Anspruch 1, wobei jeder von dem inneren Stab, dem mittleren Stab und dem äußeren Stab (240, 340, 440, 540, 840) einstückig geformt ist.

3. Teleskopartige Anordnung nach einem beliebigen der Ansprüche 1 oder 2, wobei der innere Stab ferner ein vorderes Befestigungsmittel (346) umfasst.

4. Teleskopartige Anordnung nach einem beliebigen der Ansprüche 1 bis 3, wobei die teleskopartige Anordnung eine Schulter umfasst, die in eine Bohrung einer Spritze (345, 645) passt und in Bezug auf die Spritze drehbar ist, wobei die Schulter die teleskopartige Anordnung stützt.

5. Teleskopartige Anordnung nach einem beliebigen der Ansprüche 1 bis 4, wobei der innere Stab, der mittlere Stab und der äußere Stab (240, 340, 440, 540, 840) alle in einer ersten Richtung gewindet sind, ferner umfassend eine Endkappe mit einem Gewinde in einer gegensinnigen Richtung.

6. Teleskopartige Anordnung nach einem beliebigen der Ansprüche 1 bis 5, wobei eine jeweilige Gewindesteigung von jedem von dem inneren Stab, dem mittleren Stab und dem äußeren Stab (240, 340, 440, 540, 840) eingestellt ist, um dasselbe Ausmaß an linearer Bewegung pro Umdrehung zu ergeben.

## Revendications

1. Ensemble télescopique comprenant :
une tige externe (240, 340, 440, 540, 840) comprenant
une fixation arrière (348") et
un filetage interne (329") à proximité d'une extrémité avant de celle-ci ;
une tige médiane (330) comprenant
un filetage externe (328") s'ajustant audit filetage interne de ladite tige externe (240, 340, 440, 540, 840),
**caractérisé par**
la tige médiane comprenant
une bride arrière (226', 326') pouvant coulisser à travers une partie de la tige externe (240, 340, 440, 540, 840) et étant plus large que ledit filetage interne de ladite tige externe (240, 340, 440, 540, 840) et
un filetage interne à proximité d'une extrémité avant de celle-ci ; et
l'ensemble télescopique comprenant en outre
une tige interne (320, 420) comprenant,
un filetage externe (328) s'ajustant audit filetage interne de ladite tige médiane
une bride arrière (226, 326) pouvant coulisser dans une partie de la tige médiane et étant plus large que ledit filetage interne de ladite tige médiane ;
un premier capuchon d'extrémité (324, 424) comprenant une fixation correspondante (348, 446) pour une fixation à ladite fixation arrière (346") de la tige externe (340, 440) ; et
un butoir (432) pour empêcher un dévissage de l'ensemble télescopique, ledit butoir (432) comprenant un élément d'interférence sur ledit premier capuchon d'extrémité (324, 424) pour empêcher une rotation dans une direction de contraction de ladite tige interne par rapport audit premier capuchon d'extrémité (324, 424).

2. Ensemble télescopique selon la revendication 1, dans lequel chacune desdites tige intérieure, tige médiane et tige extérieure (240, 340, 440, 540, 840) est moulée en une seule pièce.

3. Ensemble télescopique selon l'une quelconque des revendications 1 ou 2, dans lequel ladite tige interne comprend en outre une fixation de tête (346).

4. Ensemble télescopique selon l'une quelconque des revendications 1 à 3, dans lequel ledit ensemble télescopique comprend un épaulement s'ajustant dans un alésage d'une seringue (345, 645) et pouvant tourner par rapport à ladite seringue, ledit épaulement supportant ledit ensemble télescopique.

5. Ensemble télescopique selon l'une quelconque des revendications 1 à 4, dans lequel ladite tige interne, ladite tige médiane et ladite tige externe (240, 340, 440, 540, 840) sont toutes filetées dans une première orientation, comprenant en outre un capuchon d'extrémité avec un filetage dans une orientation opposée.

6. Ensemble télescopique selon l'une quelconque des revendications 1 à 5, dans lequel un pas respectif de filetage de chacune de ladite tige interne, de ladite tige médiane et de ladite tige externe (240, 340, 440, 540, 840) est ajusté pour produire la même amplitude de mouvement linéaire par rotation.
